# EUROPEAN PATENT SPECIFICATION

(11) **EP 1 012 695 B1**
(45) Date of publication and mention of the grant of the patent: **21.05.2003**
(21) Application number: 98938200.7
(22) Date of filing: 06.08.1998
(51) Int. Cl.: G06F 3/00

(54) **APPARATUS FOR GENERATING ODOR UPON ELECTRONIC SIGNAL DEMAND**
APPARAT, DER NACH ELEKTRONSICHEM SIGNAL GERÜCHE ERZEUGT
APPAREIL POUR GENERER UNE ODEUR SUR DEMANDE PAR SIGNAL ELECTRONIQUE

(30) Priority: 06.08.1997 US 907230
(43) Date of publication of application: 28.06.2000
(73) Proprietor: ILLINOIS INSTITUTE OF TECHNOLOGY, Chicago, Illinois 60616 (US)
(72) Inventor: RASOULI, Firooz, Midlothian, VA 23113 (US); ARASTOOPOUR, Hamid, Downers Grove, IL 60516 (US); OSKOUIE, Ali, Chicago, IL 60616 (US)
(74) Representative: Patentanwälte Feldmann & Partner AG
(86) International application number: US9815942
(87) International publication number: WO99008174

(56) References cited:
- EP-A- 0 123 746
- EP-A- 0 238 983
- FR-A- 2 501 468
- GB-A- 2 279 010
- US-A- 4 037 352
- US-A- 5 574 821
- US-A- 5 591 409

## Description

### BACKGROUND OF THE INVENTION

This invention relates to an apparatus for generating odor in which a controller signal, possibly from a remote location, activates a heater that heats an aroma-impregnated adsorbent.

### DESCRIPTION OF THE PRIOR ART

The Internet is a relatively new and quickly developing medium for information transfer and all forms of commerce. Internet access allows the user, through an ordinary telephone line, to view true multimedia clips, including text, pictures, audio and video. One current void on the Internet is the ability for a consumer to sample scents and aromas of food products, perfumes, flowers, wines and other products wherein the scent of the product is an important factor.

The prior art teaches several methods of distributing a specific scent on demand. The application of heat is a known method of diffusing perfumes or other odorants into the atmosphere. Pozzo, U.S. Patent 5,069,877 teaches a method and apparatus wherein heat is applied, from a source such as a lightbulb, to a perfume-impregnated heat shrink material, thus diffusing the perfume into the air. Holland, U.S. Patent 4,009,384, teaches a similar apparatus wherein heat, from a lightbulb, is applied to a porous, perfume-impregnated, temperature-resistant material.

Stern, U.S. Patent 2,562,959 teaches a system wherein compressors pump various scents through air pipes depending on an electromechanical signal generated by a film. The various scents are stored in liquid form until selected for dispersal when they are vaporized and distributed through compressed air pipe lines.

The EP0238983 discloses an automatic device for the repeated and alternating activation and diffusion of volatile purifying agent vapours. It comprises:
a) a casing having at its base an opening and in its upper part vents;
b) one porous support element disposed below the said vents and comprising several zones, each zone being impregnated with a distinct volatile purifying agent, or several porous support elements disposed below said vents, each of the said elements being impregnated with a distinct volatile purifying agent;
c) an electric heating element disposed below each of the said zones of the single porous element or below each of the said porous elements and the positioning of which is such that each heating element is sequentially and separately activated by a switch, such that once activated, it causes the air in the chamber, formed by the walls of the casing, to heat up and creates a convection movement of the air through the porous element located right above it, together with a draught from the surrounding air through the opening in the base of the housing; and
d) a sequence switch element comprising an oscillator capable of controlling the sequence consisting of a time base, and a counter associated with a logic system connected to the switches of the heating elements, so that the engagement of said elements occurs in a sequential manner according to a process providing for each heating element, alternatively, a heating period followed by a cooling period.

A device for selectively generating perfume gas is described in the GB-A-2'279'010. It includes a main body having an interior space which receives a container base having an internal space for receiving perfume containers, a selecting system able to reciprocating within the interior space of the main body, a heating system for providing heat according to the movement of the selecting system, a blast member and motors for supplying power to the blast member. The selecting system selectively exerts a force on a cap member to release a hole of the internal space from sealing and selectively activates the heating system to form a high temperature area above a predetermined surface of a wick member of said perfume container, whereby different kinds of perfume gases are freely selectively and changeably generated and released to external environment.

There is a need, however, for an apparatus that permits a user to access any number of specific scents or fragrances, specifically through a signal provided from a remote location such as an Internet server.

### SUMMARY OF THE INVENTION

It is one object of this invention to provide an apparatus which enables the user to sample various scents from a single disk.

It is another object of this invention to provide an apparatus that interfaces with a remote location and distribute selected scents based upon a predetermined signal.

It is still another object of this invention to provide an apparatus that interfaces with a personal computer to provide various scents based upon a predetermined signal.

It is yet another object of this invention to provide an apparatus that emits a selectable scent when an electric current is applied to the apparatus.

A user of the subject apparatus preferably operates a computer having a disk drive according to this invention, called a Tele-Aroma Drive (TAD). In one preferred embodiment of the subject invention, a user connects to a web site that is compatible with the subject apparatus and selects a specific scent from a computer menu. A controller, preferably contained within the disk drive generates an appropriate thermal or electrical signal to an exhaust and/or a disk containing an adsorbent. The adsorbent then disseminates the proper concentration of a scent into the user's environment.

The disk, preferably comprising a substrate and the adsorbent, is used as the means for delivery of the various scents or fragrances. In one preferred embodiment of this invention, a plurality of alternating strips of conductive material and non-conductive material are arranged on the substrate of a disk having a rectangular shape. In another preferred embodiment of the subject invention, a plurality of alternating concentric strips of conductive material and non-conductive material are arranged on the substrate of a circular disk. The adsorbent, such as a semi-porous polymer membrane, is preferably applied to the conductive material on the substrate.

A controller generates a signal, either thermal or electrical, to the disk and/or an exhaust. The controller preferably regulates the flow of electricity among power source, the disk and the exhaust. The controller preferably gathers signals from a user or a remote location such as a server, based upon predetermined characteristics of specific scent/adsorbent combinations.

A heater, such as a laser or a conductive element, heats the adsorbent depending on the specific signal received from the controller. The adsorbent, while heated, emits the specific scent from the disk. The exhaust passes a fluid, preferably air, over the adsorbent on the disk to distribute the scent emitted from the adsorbent into the environment.

The entire apparatus according to this invention is preferably housed within a disk drive, similar to a floppy disk drive used with personal computers. The disk drive can be connected to a computer with a dedicated card or through a printer port. In another preferred embodiment, a disk drive, similar to a CD-ROM drive used with personal computers, accommodates the disk formed from an arrangement of alternating concentric rings of conductive material and non-conductive material. In this preferred embodiment of this invention, the laser is used to generate heat within adsorbent.

One aspect of the invention is a system for generating odor as defined in independent claim 1.

### BRIEF DESCRIPTION OF THE DRAWINGS

The above-mentioned and other features and objects of this invention will be better understood from the following detailed description taken in conjunction with the drawings wherein:
Fig. 1 is a flowchart of the interaction among the various components of the subject invention according to one preferred embodiment of this invention.
Fig. 2 is a diagrammatic top view of a disk according to one preferred embodiment of this invention;
Fig. 3 is a diagrammatic perspective view of the apparatus according to one preferred embodiment of this invention;
Fig. 4 is a diagrammatic perspective view of the apparatus according to another preferred embodiment of this invention;
Fig. 5 is schematic view of the apparatus according to one preferred embodiment of this invention; and
Fig. 6 is a diagrammatic cross-sectional side view of a membrane module according to a comparative example.

### DESCRIPTION OF THE PREFERRED EMBODIMENTS

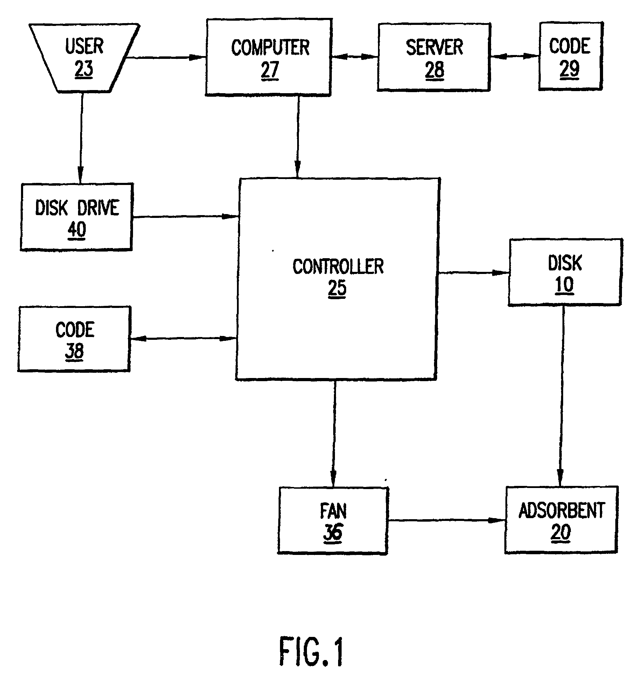

Fig. I shows a flowchart of the interaction among the various components of the apparatus according to one preferred embodiment of this invention. As shown in Fig. 1, user 23 of the subject apparatus preferably operates computer 27 with disk drive 40, also called a Tele-Aroma Drive or TAD.

In one preferred embodiment of the subject invention, user 23 connects from computer 27 to a web site on server 28 that is compatible with the subject apparatus. User 23 selects a specific scent from a menu, such as a perfume. Server 28 receives the signal from computer 27 and consults server code 29 for the proper signal to transmit back to computer 27. Server 28 transmits a return signal back to computer 27 based upon parameters of the selected perfume. This return signal is decoded by controller 25, which is preferably but not necessarily contained within disk drive 40. Controller 25 consults controller code 38 to generate the appropriate thermal or electrical power to fan 35 and/or disk 10 containing adsorbent 20. Given the proper power, adsorbent 20 disseminates the proper concentration of scent into an environment surrounding user 23.

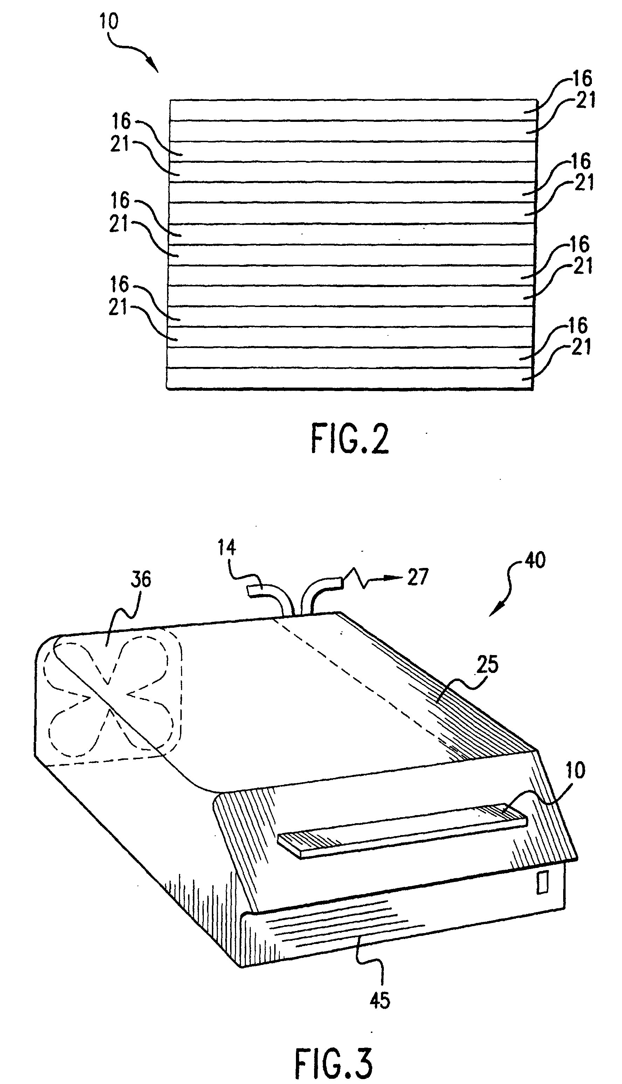

An apparatus for generating odor utilizes disk 10 such as that shown in Fig. 2. Disk 10 preferably comprises two basic components: a substrate and adsorbent 20. Substrate, as used in this specification, is a generic term referring to the basic composition or support material of disk 10. Substrate material is preferably a polymer or other suitable non-conductive material.

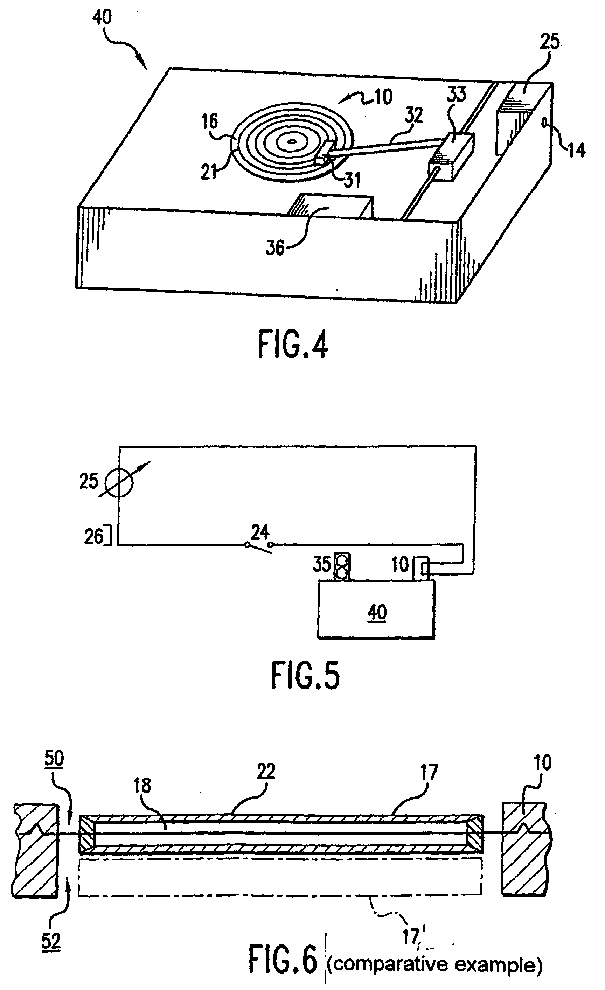

In one preferred embodiment of this invention shown in Fig. 2, a plurality of alternating strips of conductive material 16 and non-conductive material 21 are arranged on the substrate of disk 10 having a rectangular shape. Fig. 4 shows another preferred embodiment of the subject invention in which a plurality of alternating concentric strips of conductive material 16 and non-conductive material 21 are arranged on the substrate of disk 10 having a circular shape.

Conductive material 16 may be a highly conductive metal such as aluminum or copper, or a conductive non-metal such as carbon. The strips or rings of conductive material 16 are preferably connected together with non-conductive material 21 to allow the strips of conductive material 16 to heat up or accept an electrical current independently of other strips of conductive material 16.

Adsorbent 20, as used in this specification, is a generic term referring to material that adsorbs desired odor-producing chemicals, such as aroma solutions and perfumes. The function of adsorbent 20 is to adsorb the odor-producing chemical when treated and desorb the odor-producing chemical when a stimulus, such as heat or high temperature air flow, is applied.

Adsorbent 20 is selected based upon several important criteria. Adsorbent 20 must have reasonable permeability. It is preferable that the characteristics of adsorbent 20 allow for the transport of aroma solution. Adsorbent 20 must also have a high capacity for the aroma solution. An ideal adsorbent 20 should retain a large amount of aroma solution per unit volume of adsorbent 20. Adsorbent 20 should also possess indiscriminate permeability to all ingredients of the aroma solution. An ideal adsorbent 20 will pass all components of the aroma solution evenly. Adsorbent 20 should also be inert such that no chemical interaction takes place between adsorbent 20 and the aroma solution. Adsorbent 20 should not desorb the aroma solution at temperatures under 100°F. Finally, adsorbent 20 should possess a resistance to high and variant temperatures, between 120°F and 500°F, and cyclical heating and reheating such that adsorbent 20 characteristics do not change.

In one preferred embodiment of this invention, adsorbent 20 is applied to conductive material 16 on the substrate. Adsorbent 20 may be a gel, a paste or any other material that exhibits the characteristics described above. Other possible adsorbent 20 materials include: packing materials used in chromatography such as Chromosorb 101™, Porpak Q™, Apiezon L™, Carbowax20 M™, OV-210™, and/or Dexsil 300 GC™; inorganic materials such as silica gel, activated carbon, carbon fiber, and/or zeolites; synthetic polymers and responsive polymeric materials that exhibit changes in properties in response to a control variable such as temperature or light; and/or organic compounds such as cellulose compounds, waxes or natural pastes made with mixtures of finely sifted sawdust and syrup of gum arabic.

In a preferred embodiment of this invention, adsorbent 20 is a semi-porous membrane 22. Specifically, such materials as Teflon, Tygon, silicon rubber or other polymers may be used as adsorbent 20. Silicon rubber demonstrates favorable results according to the desired characteristics listed above for an ideal adsorbent 20. In the embodiment of this invention wherein adsorbent 20 is a polymer such as semi-porous membrane 22, adsorbent must be treated in one of several methods to suspend the aroma solution or fragrance within pores of adsorbent 20. In an alternate preferred embodiment of this invention, key chemical ingredients can be mobilized in adsorbent 20, and by selective precise heating of combinations of such key chemical ingredients, various odors can be generated in-situ.

As shown by the flow chart in Fig. 1, controller 25 generates a signal to an input of the apparatus. The signal is dependent upon specifications or characteristics entered by user 23 from personal computer 27 or by a vendor from server computer 28. Controller 25 preferably regulates the flow of electricity among power source (not shown in Fig. 1), disk 10 and exhaust 35. As discussed above, controller 25 also gathers signals from a user or a remote location such as server 28 shown in Fig. 1 and, based upon predetermined characteristics of specific aroma solutions and adsorbents 20, generates an input, either thermal, mechanical or electrical, to disk 10 and/or exhaust 35.

Heater, such as laser 31 shown in Fig. 4 or conductive element 18 shown in Fig. 6, heats adsorbent 20 depending on the specific signal received from controller 25. Heater reaches a predetermined temperature based upon a signal received from controller 25. The predetermined temperature is calculated based upon the chemical properties of the aroma solution to be distributed as well as the characteristics of the specific adsorbent 20. Each aroma sotution/adsorbent 20 combination may require a slightly different temperature to effectively separate the scent from adsorbent 20. In one comparative example heater is conductive element 18, such as that shown in Fig. 6 and discussed below. When a current is applied to conductive element 18, a desired temperature is attained with slight variations of the applied current.

A predetermined temperature is achieved by controlling the time of heating instead of the temperature of conductive element 18. Timed heating thus eliminates the need for a thermocouple connected to conductive element 18.

In another preferred embodiment of this invention, shown in Fig. 4, laser 31 is used to generate heat within adsorbent 20. If laser 31 is used as heater, an arrangement of alternating conductive material 16 and non-conductive material 21 is unnecessary. Like a CD-ROM disk, disk 10 in this preferred embodiment can be fabricated entirely from a non-conductive substrate, and adsorbent 20 can be arranged in concentric rings on such non-conductive substrate.

In yet another preferred embodiment of this invention, heater may comprise a blower producing a high temperature, high temperature air flow. In this embodiment, high temperature gas, such as air, is discharged across the surface of adsorbent 20 to generate heat in adsorbent 20.

Adsorbent 20, while heated or exposed to a high-temperature, high-velocity gas, emits the specific aroma from disk 10. According to one preferred embodiment of this invention, exhaust 35, such as fan or blower 36, passes a fluid, preferably air, over adsorbent 20 on disk 10 and preferably through vents 45 in disk drive 40. In another preferred embodiment of this invention, exhaust 35 may discharge a high temperature air flow which initiates fast evaporation or sublimation of the aroma solution on adsorbent 20. Exhaust 35 preferably distributes the scent emitted from adsorbent 20 into the environment surrounding user 23, enhances adsorption of the scent, and cools heater 30 and adsorbent 20 to slow dissipation of further scent when a new scent is selected or the current scent is satisfactorily distributed. The combined distribution and cooling action of exhaust 35 avoids mixing multiple fragrances during extended use of the apparatus.

In an embodiment according to a comparative example, adsorbent 20 such as at least one membrane module 17 having semi-porous membrane 22, as shown in Fig. 6, is moveable from a first position to a second position within apparatus, such as disk 10. In this preferred embodiment, membrane module 17, when selected, moves from a first position in upper compartment 50 to a second position in lower compartment 52. Lower compartment 52 is preferably positioned within a discharge path of exhaust 35. This preferred embodiment of this invention prevents excess mixing of scents of the selected membrane module 17' with unselected membrane modules 17. Controller 25 preferably controls the movement of adsorbent 20 such as membrane module 17, 17' from a first position to a second position and vice versa.

The entire apparatus according to this invention is preferably housed within disk drive 40, much like a stagnant 3.5" floppy disk drive. Disk drive 40 is preferably not larger than a typical computer speaker, and may be purchased by a consumer like any other personal computer peripheral device. Disk drive 40 can be connected to computer 27 with a dedicated card or through a printer port to input 14 of disk drive 40. Disk 10 is preferably inserted into disk drive 40 which is configured with computer 27.

In one preferred embodiment of this invention shown in Fig. 3, disk drive 40 accepts disk 10 such as that shown in Fig. 2. In disk drive 40 shown in Fig. 3, controller 25 applies various currents and/or thermal energy to strips of conductive material 16 or a matrix of conductive elements 18, such as those in membrane module 22 shown in Fig. 6.

Another preferred embodiment of this invention is shown in Fig. 4, wherein disk drive 40 having input 14 accommodates disk 10 formed from an arrangement of alternating concentric rings of conductive material 16 and non-conductive material 21. Disk drive 40 shown in Fig. 4 is configured similar to a CD-ROM drive used with personal computers. Disk drive 40 comprises actuator motor 33 which controls actuator arm 32 to position laser 31 at a predetermined radius of disk 10. A disk motor (not shown) rotates disk 10 so that laser 31 heats a specific concentric ring preferably layered with adsorbent 20.

The present example is to be considered comparative.

### EXPERIMENTAL PROCEDURE

An experimental apparatus was developed to test different polymeric membranes for permeation of odor. A schematic of the experimental apparatus is shown in Fig. 5. In one preferred embodiment of the subject invention, controller 25 comprises a variable transformer (VARIAC) for distributing the appropriate currents to input 14 of disk drive 40.

In one embodiment of a comparative example, disk 10 comprises six pairs of inputs electrically connected with six corresponding membrane modules 17 arranged in a row. Each membrane module 17 in this experimental embodiment comprises conductive element 18 surrounded with semi-porous membrane 22. For the purposes of this experiment, semi-porous membrane 22 is formed into a tube surrounding conductive element 18.

Several polymeric materials including Teflon, Tygon and silicon rubber were tested for use as semi-porous membrane 22. Silicon rubber was chosen for this experiment based upon its beneficial characteristics. The silicon rubber was formed into tubes, having an internal surface and an external surface, for use as semi-porous membrane 22 of membrane module 17. For this experiment, silicon rubber tubing approximately 2 inches in length were used as semi-porous membrane 22. Various thicknesses of semi-porous membrane 22 were investigated.

Appropriate aroma solutions, in this case various commercially available perfumes, were injected, each in a separate membrane module 17, between the annulus formed between conductive element 18 and semi-porous membrane 22. Aroma solution was drained from semi-porous membrane 22 after an appropriate soaking time, in this case approximately 48 hours. Alternatively, aroma solution may remain within semi-porous membrane 22. In another preferred embodiment, the external surface of semi-porous membrane 22 was soaked with aroma solution. In this experiment, each membrane module 17 was filled with 0.2 ml of perfume.

Each membrane module 22 was assembled using conductive element 18 made with 24 BNC Nickel-Chrome resistance wire. Conductive element 18 was fed through the center of semi-porous membrane 17 and each end of semi-porous membrane 22 was sealed using a fast-setting aluminum epoxy. For this experiment, a Teflon sleeve was applied between conductive element 18 and semi-porous membrane 17 to prevent direct contact of the hot conductive element 18 with the semi-porous membrane 17.

Membrane modules 22 containing semi-porous membranes 17 treated with the aroma solution were then connected to power source 26. For experimental purposes, a 110 Volt power source 22 was transformed into approximately 3-4 Volts prior to application to membrane module 17.

Fan 36 with a flow rate of about 32 cfm was used as exhaust 35 to disseminate the fragrance of the aroma solution. In one comparative experimental embodiment of this invention, air flow from fan 35 was directed over an exposed upper surface of membrane modules 17. In another, alternate comparative embodiment air flow was directed beneath the surface of membrane modules 17 through slots or compartment 52, shown in Fig. 6, constructed under each membrane module 17.

In yet another embodiment of this invention, shown in Fig. 6, membrane module 17 is moveable from a first position within upper compartment 50 to a second position in lower compartment 52. In this preferred embodiment of the invention, selected membrane module 17' is positioned in the path of air flow from fan 35 (not shown in Fig. 6).

A K-type thermocouple measured the surface temperature of each conductive element 18. The resulting electric signal was then recorded using a Strawberry Tree™ data acquisition and control system on a personal computer. The temperature of conductive element 18 was then controlled by the same system. The optimum temperature of conductive element 18, that is, the highest temperature possible without altering the properties of the aroma solution or damaging membrane module 17 was obtained by trial and error.

The present experimental results are to be considered comparative, unless specified otherwise.

### EXPERIMENTAL RESULTS

Two air flow paths were tested, over the upper, exposed surface of membrane modules 17 and beneath the surface of membrane modules 17. Experimental results revealed that air flow over the upper surface of membrane module 17 results in better dispersal of the scent generated by membrane module 17. Directing the air beneath the surface of membrane modules 17 results in scents from differently scented membrane modules 17 intermingling. Membrane module 17' positioned in the path of air flow from fan 35 greatly reduced the mixing of scents from the various unselected membrane modules 17.

Tests also revealed that, to avoid mixing different scents among membrane modules 17, a delay of at least 15 seconds is necessary between the deactivation of one membrane module 17 and the activation of a different membrane module 17. However, such a delay was unnecessary in the embodiment of this invention wherein membrane module 17' is moveable to a second position.

Test results revealed that the thickness of semi-porous membrane 22 was proportional to the time lag for detection of the scent of membrane module 17. Experimental results also revealed that soaking only the inner surface of the semi-porous membrane 22 results in better dissemination of the scent from membrane module 17. Membrane module 17 could be reused many times before requiring replacement and/or reapplication of aroma solution.

Because each perfume has different characteristics, the required temperature of conductive element 18 was different for each brand of perfume. This suggests that the selection of the temperature should be done automatically when the type of aroma solution, such as a brand of perfume, is specified.

While in the foregoing specification this invention has been described in relation to certain preferred embodiments thereof, and many details have been set forth for purposes of illustration, it will be apparent to those skilled in the art that the apparatus is susceptible to additional embodiments and that certain of the details described herein can be varied considerably without departing from the basic principles of the invention.

## Claims

1. A system for generating odor, the system comprising:
an apparatus for containing a substrate;
a controller separate from the apparatus generating a signal on demand, an input of the apparatus accepting the signal;
a heater (31) simultaneously heating one or more adsorbents of a plurality of adsorbents (20) upon demand as a function of the signal, each adsorbent (20) retaining a releasable aroma; **characterized in that**
a plurality of adsorbents (20) are deposited in a fixed position on the substrate, wherein a plurality of alternating strips or concentric rings of a conductive material (16, 18) and a non-conductive material (21) are arranged on the substrate and adapted to allow to heat up one or more of the adsorbents (20); and
the system comprising an exhaust (35, 36) discharging a fluid over the one or more adsorbents (20).

2. The apparatus of claim 1 wherein the at least one adsorbent is deposited on the conductive material.

3. The apparatus of claim 1 wherein the at least one adsorbent is a polymer membrane.

4. The apparatus of claim 1 wherein the at least one adsorbent is moveable from a first position to a second position.

5. The apparatus of claim 1 wherein the heater comprises a conductive element in communication with an electric current supply.

6. The apparatus of claim 1 wherein the heater comprises a laser.

7. The apparatus of claim 1 wherein the controller comprises a computer.

8. The apparatus of claim 1 wherein the exhaust comprises a blower.

9. The apparatus of claim 1 wherein the exhaust discharges fluid over the at least one adsorbent as a function of the signal.

## Patentansprüche

1. Ein System zum erzeugen von Düften, wobei das System umfasst:
einen Apparat zum Beinhalten eines Substrates;
einen vom Apparat separaten Controller, der ein Signal bei Bedarf generiert, einen Eingang des Apparates, der das Signal akzeptiert;
eine Heizeinrichtung (31) welche bei Bedarf als Funktion des Signals gleichzeitig eines oder mehrere adsorbierende Mittel aus einer Vielzahl von adsorbierenden Mittel erwärmt, wobei jedes adsorbierende Mittel ein freisetzbares Aroma speichert;
**dadurch gekennzeichnet, dass**
eine Vielzahl von adsorbierenden Mittel (20) in einer festgelegten Position auf dem Substrat deponiert sind, wobei eine Vielzahl von alternierenden Streifen oder konzentrischen Ringen eines leitenden Materials (16, 18) und eines nicht-leitenden Materials (21) auf dem Substrat angeordnet und so angepasst sind, so dass sie ein Aufheizen eines oder mehrerer der adsorbierende Mittel (20) erlauben;
und dass das System einen Lüfter (35, 36) umfasst, der ein Fluid über das eine oder die mehreren adsorbierenden Mittel ausstösst.

2. Der Apparat gemäss Anspruch 1, worin das wenigstens eine adsorbierende Mittel auf dem leitenden Material angeordnet ist.

3. Der Apparat gemäss Anspruch 1, worin das wenigstens eine adsorbierende Mittel eine Polymermembran ist.

4. Der Apparat gemäss Anspruch 1, worin das wenigstens eine adsorbierende Mittel beweglich von einer ersten Position in eine zweite Position bringbar ist.

5. Der Apparat gemäss Anspruch 1, worin die Heizeinrichtung ein leitendes Element in Verbindung mit einer elektrischen Spannungsquelle umfasst.

6. Der Apparat gemäss Anspruch 1, worin die Heizeinrichtung einen Laser umfasst.

7. Der Apparat gemäss Anspruch 1, worin der Controller einen Computer umfasst.

8. Der Apparat gemäss Anspruch 1, worin der Lüfter ein Gebläse umfasst.

9. Der Apparat gemäss Anspruch 1, worin der Lüfter Fluid über das wenigstens eine adsorbierende Mittel als eine Funktion des Signals ausstösst.

## Revendications

1. Système destiné à générer une odeur, le système comprenant :
un dispositif destiné à contenir un substrat,
un contrôleur séparé du dispositif générant un signal à la demande, une entrée du dispositif recevant le signal,
un dispositif de chauffage (31) chauffant simultanément un ou plusieurs adsorbants parmi une pluralité d'adsorbants (20) à la demande en fonction du signal, chaque adsorbant (20) conservant un arôme pouvant être libéré,
**caractérisé en ce que**
une pluralité d'adsorbants (20) sont déposés à une position fixe sur le substrat, où une pluralité de bandes alternées ou d'anneaux concentriques d'un matériau conducteur (16, 18) et d'un matériau non-conducteur (21) sont disposés sur le substrat et conçus pour permettre de chauffer un ou plusieurs des adsorbants (20), et
le système comprenant un échappement (35, 36) délivrant un fluide sur les un ou plusieurs adsorbants (20).

2. Dispositif selon la revendication 1, dans lequel le au moins un adsorbant est déposé sur le matériau conducteur.

3. Dispositif selon la revendication 1, dans lequel le au moins un adsorbant est une membrane de polymère.

4. Dispositif selon la revendication 1, dans lequel le au moins un adsorbant est mobile d'une première position vers une seconde position.

5. Dispositif selon la revendication 1, dans lequel le dispositif de chauffage comprend un élément conducteur en communication avec une alimentation en courant électrique.

6. Dispositif selon la revendication 1, dans lequel le dispositif de chauffage comprend un laser.

7. Dispositif selon la revendication 1, dans lequel le contrôleur comprend un ordinateur.

8. Dispositif selon la revendication 1, dans lequel l'échappement comprend un ventilateur.

9. Dispositif selon la revendication 1, dans lequel l'échappement délivre un fluide sur le au moins un adsorbant en fonction du signal.
